# EUROPÄISCHE PATENTSCHRIFT

(11) **EP 3 113 820 B1**
(45) Veröffentlichungstag und Bekanntmachung des Hinweises auf die Patenterteilung: **14.03.2018**
(21) Anmeldenummer: 15716714.9
(22) Anmeldetag: 19.02.2015
(51) Int. Cl.: A61M 16/04, A61M 16/08

(54) **TRACHEALKANÜLENINNENROHR**
TRACHEAL CANNULA INNER TUBE
TUBE INTÉRIEUR DE SONDE TRACHÉALE

(30) Priorität: 06.03.2014 DE 102014003362
(43) Veröffentlichungstag der Anmeldung: 11.01.2017
(73) Patentinhaber: Primed Halberstadt Medizintechnik GmbH, 38820 Halberstadt (DE)
(72) Erfinder: RAMDOHR, Bastian, 38820 Halberstadt (DE); TRÄGER, Peter, 38820 Halberstadt (DE); KLIMENTA, Klaus, 38835 Zilly (DE)
(74) Vertreter: Donath, Dirk
(86) Internationale Anmeldenummer: PCT/DE2015/000088
(87) Internationale Veröffentlichungsnummer: WO 2015/131867

(56) Entgegenhaltungen:
- EP-A1- 0 107 779
- EP-A1- 1 479 405
- US-A- 4 033 353
- US-A1- 2013 269 690
- US-A1- 2014 034 048

## Beschreibung

Die vorliegende Erfindung betrifft ein Trachealkanüleninnenrohr zum Einführen in ein Trachealkanülenaußenrohr einer Trachealkanüle zum Einsetzen in ein Tracheostoma.

Bei größeren operativen Eingriffen im oberen Atemtrakt kann das Anlegen einer künstlichen Atemöffnung im Halsbereich (s.g. Tracheostoma) notwendig sein, so dass die Luft unter Umgehung von Mundraum und Kehlkopf direkt in die Lunge eingeatmet wird.

Bei der Laryngektomie wird dieses Stoma des Patienten dauerhaft offen gehalten, indem eine Trachealkanüle zum Stabilisieren und Offenhalten der neuen Atemöffnung eingesetzt wird.

Trachealkanülen (auch Tracheostoma- Prothesen oder Tracheostoma-Tuben genannt) zur Behandlung kehlkopfloser (laryngektomierter) oder tracheostomierter Patienten mit eröffneter Kehle (s.g. Tracheostoma) sind seit Jahrzehnten bekannt. Die Trachealkanülen werden dabei in das Tracheostoma eingesetzt und befestigt.

Trachealkanülen bestehen zumeist aus einer rohr- oder schlauchförmigen Außenkanüle für die Hindurchführung durch einen Hals- und Luftröhrenschnitt in die Luftröhre eines Patienten, in welche eine ebenfalls schlauchförmige Innenkanüle führbar und mit der Außenkanüle verriegelbar ist, und einem an der Außenkanüle beweglich angebrachten Kanülenschild, durch welches hindurch die Außenkanüle greift, zur Anlage am Hals, durch dessen Anlage am Hals des Patienten die Position der Außenkanüle bzw. des Endes der Außenkanüle in der Luftröhre des Patienten definiert wird.

Trachealkanülen, die aus einer Außenkanüle und einer Innenkanüle bestehen, sind zumeist als bogenförmige Kanülen ausgeführt, da sich diese Bogenform besonders gut für die bei der Tracheostomie gegebene Anatomie des Tracheostomas zum Einführen der Trachealkanülen in das Tracheostoma eignet.

Aus der DE 10 2005 030 300 A1 ist eine bogenförmige Trachealkanüle mit beweglichem Schild bekannt, die eine freie Schwenk- und Drehbarkeit in sämtliche Raumrichtungen, [um eine Raumachse (X) drehbar und um zwei Raumachsen (Y, Z) beliebig verschwenkbar] ermöglicht und auch bei Dauerbelastung eine hohe mechanische Stabilität aufweist.
Dabei ist die Kanüle gegenüber dem Kanülenschild speziell gelagert, in dem die Kanüle über ein Kugelgelenk formschlüssig mit einer Gelenkpfanne des Kanülenschilds verbunden ist.

Aus der DE 20 2004 020 108 U1 ist ein Tracheostomainnenrohr mit einem Kombinationsadapter bestehend aus einem Tracheostomarohr, das an seinem distalen Ende einen festen Adapteransatz trägt, der an seinem, dem Tracheostomarohr abgewanden Ende einen weichen Adapter besitzt, der mit dem Adapteransatz verbunden ist, bekannt.

Die DE 20 2004 020 109 U1 offenbart ein Tracheostomarohr mit einem festen Adapter (Konnektor) bestehend aus einem Tracheostomarohr, das an seinem distalen Ende einen Adapter (Konnektor) trägt, wobei der Adapter (Konnektor) an seinem freien Ende zumindest eine Ausnehmung in seinem Rand besitzt.

Eine Trachealkanüle mit einem festem Konnektor ist bspw. auch durch das eingetragene Design DE 40 2011 006 509.6 bekannt.

Der Nachteil der bekannten Trachealkanüleninnenrohre mit festem Adapter (Konnektor) besteht darin, dass ein mit dem Adapter (Konnektor) verbundenes Bauteil, bspw. ein Beatmungsschlauch, nicht leicht um die Raumachse -X-X- [= Symmetrieachse des Adapters (Konnektors)] gedreht werden kann, so dass eine durch Verdrehung des Bauteils gegenüber der Trachealkanüle bestehende Spannung zwischen diesen beiden Teilen nicht beseitigt werden kann.

US 4 009 720 A offenbart einen dichtenden Klemmkonus für den Verschluss einer Tracheostomiekanüle mit innerer und äußerer Kanüle. Dieser einstellbare, dichtende Verschluss befindet sich entweder zwischen der inneren und der äußeren Kanüle oder zwischen der inneren Kanüle. Dabei ist dieser Verschluss / Kopplungsmechanismus komplex aufgebaut und umfasst u.a. einen äußeren und einen inneren Arretierungsmechanismus, wobei die äußere Arretierung durch den drückenden Zugriff von zwei Fingern lösbar ist, sowie einen inneren Dichtring, dessen Bund der Kegelstumpf-artig geformte Enden aufweist, die vom Ring weg in entgegengesetzte Richtung auslaufend sind. Der Dichtring liegt dabei zwischen Innenkanüle und Außenkanüle bzw. Konnektor, wobei die Arretierung der Verbindung der beiden Bauteile durch zwei Halteelemente zwischen Innenkanüle und Außenkanüle bzw. Konnektor in der Art und Weise generiert ist, dass deren eingerastete Position der Wirkung des Kegelstumpf-artig ausgeformten Dichtrings entlang der Längsachse des Verschlusses kräftemäßig entgegen wirkt. Dadurch entsteht eine dichte und drehbare Verbindung zwischen Innenkanüle und Außenkanüle bzw. Konnektor, die durch das komplexe Zusammenwirken von drei Ring- oder Nutbereichen erreicht wird.

US 4 033 353 A offenbart eine Tracheostomiekanüle mit innerer und äußerer Kanüle, wobei ein Schlauch, durch welchen Gas leitbar ist, entlang der Außenkanüle zu einem elastischen Ballon führt und die Außenkanüle starr mit dem Schild der Tracheostomiekanüle verbunden ist. Die Verbindung der zwischen der Innen- und der Außenkanüle ist dabei beweglich gestaltet, in dem die Innenkanüle durch einen komplexen Verriegelungsmechanismus in der Außenkanüle gehaltert ist. Dazu wirken an der Verbindungsstelle zwischen Innen- und Außenkanüle eine Haltekralle an einer Haltekante der Außenkanüle, an welcher sich die Kralle abstützt und eine Haltekralle an der Innenkanüle, welche sich in gegenläufiger Richtung an einer Haltekante der Außenkanüle abstützt, mit ihrer Kraftwirkung in entgegengesetzter Orientierung. Dadurch werden beide Bauteile miteinander beweglich verspannt.
Die Dichtheit dieser beweglichen Verbindungsstelle wird durch einen konisch verlaufenden Dichtring der Innenkanüle, welcher sich an der Außenkanüle abstützt, generiert (ähnlich der technischen Lösung der US 4 009 720 A). Durch dieses Zusammenspiel von zwei Halteelementen und einem Dichtungselement wird eine bewegliche und dabei dichte Verbindung zwischen Innen und Außenkanüle geschaffen.

In der DE 195 14 433 C1 wird eine Tracheostomiekanüle zum Einsatz in ein Tracheostoma beschrieben, welche eine schlauchförmige Außenkanüle, in die eine ebenfalls schlauchförmige Innenkanüle führbar und mit der Außenkanüle am proximalen Teil verriegelbar ist, um ein Kanülenrohr zu bilden, aufweist, wobei im proximalen Bereich der Außenkanüle ein Kanülenschild zur Anlage am Hals angebracht ist, durch welches hindurch der proximale Teil der Außenkanüle greift. Dabei ist das Kanülenrohr gegenüber dem Kanülenschild um wenigstens zwei Raumachsen (Y, X) verschwenkbar gelagert, wobei die Verschwenkbarkeit um Y durch einen um die Außenkanüle greifenden und an dieser drehgelagerten Ring und die Verschwenkbarkeit um X durch die Drehlagerung des Kanülenschilds an dem Ring generiert wird.

US 2010 / 0 307 488 A1 offenbart eine Tracheostomiekanüle, bei der u.a. die innere Kanüle mit einer passend ausgestalteten Hülse versehen ist, wobei ein Schnappverschluss zwischen beiden Bauteilen in der Art ausgebildet ist, dass die Innenkanüle einen Kragen / Bund aufweist, gegen den sich die Hülse abstützen kann, wobei das Ende der Innenkanüle für eine entgegengesetzt orientierte zweite Abstützstruktur oberflächenstrukturiert ist. Durch dieses Zusammenspiel von zwei gegenläufig verspannenden Halteelementen eines Schnappverschlusses wird eine bewegliche Verbindung zwischen der Innenkanüle und der Hülse geschaffen.

Durch diese technischen Lösungen ist grundsätzlich eine Verdrehung von Kanüle oder Adapter möglich. Der Nachteil dieser bekannten Lösungen besteht jedoch darin, dass sie komplex ausgestaltet sind, so dass sie nicht leicht herstellbar und handhabbar sind, um bspw. einen Beatmungsschlauch leicht um die Raumachse -X-X- [= Symmetrieachse des Adapters (Konnektors)] drehen zu können, so dass eine durch Verdrehung des Bauteils gegenüber der Trachealkanüle bestehende Spannung zwischen diesen beiden Teilen vermieden werden kann.

Die EP 1 479 405 A1 offenbart eine Schnellverschlussverbindung für einen Luftröhrenkatheter, die einen männlichen Endabschnitt, der am Vorderende des Luftröhrenkatheter angeordnet ist, mit einem weiblichen Endabschnitt verbindet, der an einem Vorderende eines Anschlusses zur künstlichen Beatmung bereitgestellt ist, wobei der männliche Endabschnitt eine konische äußere Randeingriffsfläche umfasst, die um den Außenrand des männlichen Endabschnitts herum ausgebildet ist, und einen ringförmigen konvexen Abschnitt, der wie ein Flansch entlang einer hinteren Kante der äußeren Randeingriffsfläche herausragt.
Der weibliche Endabschnitt umfasst bevorzugt eine konische innere Randeingriffsfläche, die um einen Innenrand des weiblichen Endabschnitts herum ausgebildet ist und mit Druck in die äußere Randeingriffsfläche eingreift. Zudem weist der weibliche Endabschnitt bevorzugt einen externen Zylinder auf, der um den Außenrand des weiblichen Endabschnitts herum eingebaut und aus einem elastischen und flexiblen Material ausgebildet ist.
Werden der männliche Endabschnitt und der weibliche Endabschnitt so miteinander verbunden, dass der erstgenannte in den letztgenannten eingesetzt wird, so sitzt die innere Randeingriffsfläche des weiblichen Endabschnitts mit einem Presssitz auf der äußeren Randeingriffsfläche. Dabei rastet jede der Sperrklinken, die auf dem zugehörigen zweiten gegenüberliegenden Wandabschnitt des externen Zylinders vorhanden sind, in die rückwärtige Seitenwand des ringförmigen konvexen Abschnitts des zylindrischen Teils ein. Dadurch wird verhindert, dass der männliche Endabschnitt aus dem weiblichen Endabschnitt herausrutscht.

Der Nachteil dieser technischen Lösung besteht darin, dass eine Vielzahl verschiedenster Elemente aufwendig ineinander gefügt ist, um eine Verbindung von männlichem und weiblichem Endstück zu gewährleisten.

Die US 2014/0034048 A1 offenbart einen Trachealtubus der eine äußere Kanüle, ein Flanschelement zur Befestigung der äußeren Kanüle und eine innere Kanüle umfasst, wobei ein äußerer Kanülenanschluss an dem proximalen Ende der äußeren Kanüle vorgesehen ist und die innere Kanüle innerhalb der äußeren Kanüle angeordnet ist. Die innere und die äußere Kanüle sind dabei koaxial, wobei die innere Kanüle einen komprimierbaren proximalen Bereich aufweist, der innerhalb des äußeren Kanülenanschlusses positioniert ist, und die äußere und die innere Kanüle miteinander verriegelbar sind.

Der Nachteil dieses Trachealtubus besteht darin, dass die äußere und die innere Kanüle nicht gegeneinander gedreht werden können.

Der Erfindung liegt daher die Aufgabe zugrunde, ein alternatives Trachealkanüleninnenrohr anzugeben, welches die Nachteile des Standes der Technik vermeidet, insbesondere eine aufwandgeringere Verbindung zwischen einem Trachealkanüleninnenrohr und einem Adapter bereit stellt, die gegeneinander verdrehbar sind, und dabei verhindert, dass der Endabschnitt des Trachealkanüleninnenrohrs aus dem Endabschnitt des Adapters herausrutscht, so dass bspw. ein über den Adapter mit dem Trachealkanüleninnenrohr verbundener Beatmungsschlauch entlastet sowie vor dem Verdrehen bewahrt werden kann, und auch bei Dauerbelastung eine hohe mechanische Stabilität dieser Verbindung aufrecht erhalten wird.

Gelöst wird diese Aufgabe durch ein Trachealkanüleninnenrohr gemäß dem ersten Patentanspruch. Vorteilhafte Ausgestaltungen sind in den nachgeordneten Ansprüchen ausgeführt.

Das Wesen der Erfindung besteht darin, dass die bogenförmige Röhre des Trachealkanüleninnenrohrs sich an ihrem einen freien Ende über einen Bereich konisch erweitert, welcher anschließend in einen gerade ausgeformten Rohrbereich übergeht, so dass der Durchmesser des gerade ausgeformten Rohrbereichs größer ist als der Durchmesser des Krümmungsbereiches und dass an diesem gerade ausgeformten Rohrbereich ein um die Achse -X-X- um 360° drehbar gelagertes, hülsenförmiges Adapterstück angebunden ist, in dem der gerade ausgeformte Rohrbereich in seiner äußeren Oberfläche eine umlaufende Nut aufweist, in welche passgenau eine umlaufende Wulst der inneren Oberfläche des Adapterstücks eingreift, so dass ein durch Nut und Wulst geführtes Reiblager (Dreh-Gleitlager) zwischen der äußeren Oberfläche des gerade ausgeformten Rohrbereichs und der inneren Oberfläche des Adapterstücks, welche sich zueinander in einem leicht berührenden Oberflächenkontakt befinden, ausgebildet ist.

Die Erfindung wird nachstehend an Hand des Ausführungsbeispiels und der Figuren näher erläutert. Dabei zeigt:
- Fig. 1:: eine schematische Übersichtsdarstellung einer Ausführungsform des erfindungsgemäßen Trachealkanüleninnenrohrs und
- Fig. 2:: eine Schnittdarstellung durch das Trachealkanüleninnenrohr gemäß Fig. 1.

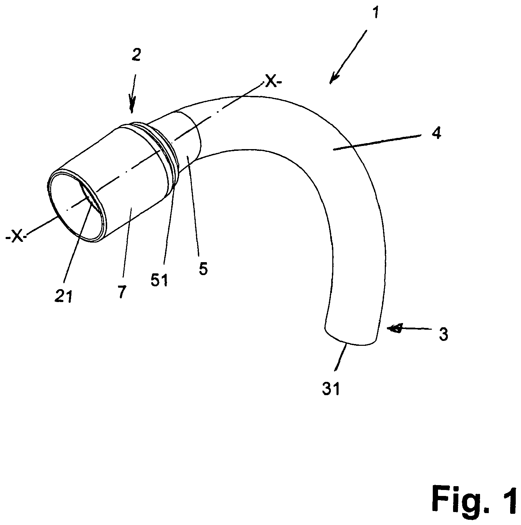

Das in Fig. 1 dargestellte Trachealkanüleninnenrohr zum Einführen in ein Trachealkanülenaußenrohr einer Trachealkanüle besteht aus einer rohrförmigen Kanüle (1) mit einer ersten Öffnung (21) an einem ersten freien Ende (2) und einer zweiten Öffnung (31) an einem zweiten freien Ende (3).

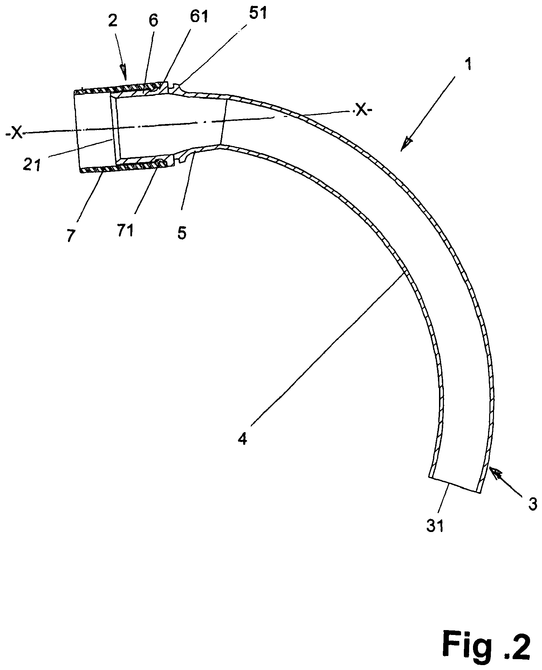

Diese Kanüle (1) weist, wie in Fig. 2 dargestellt, zwischen den beiden freien Enden (2 und 3) einen bogenförmigen Krümmungsbereich (4) auf.

Dieser Krümmungsbereich (4) geht am ersten freien Ende (2) in einen sich konisch aufweitenden Rohrbereich (5) über, an den sich ein gerade ausgeformter Rohrbereich (6) unmittelbar anschließt.

Der Durchmesser des gerade ausgeformten Rohrbereichs (6) ist dadurch größer als der Durchmesser des Krümmungsbereiches (4), bspw. in einem Verhältnis von 1,5 : 1 (je nach Größe der Kanüle).

An dem gerade ausgeformten Rohrbereich (6) ist ein um die Drehachse -X-X- um 360° drehbar gelagertes, hülsenförmiges Adapterstück (7) angebunden, in dem der gerade ausgeformte Rohrbereich (6) in seiner äußeren Oberfläche eine umlaufende Nut (61) aufweist, in welche passgenau eine umlaufende Wulst (71) der inneren Oberfläche des Adapterstücks (7) eingreift, so dass ein durch Nut (61) und Wulst (71) geführtes Reiblager (Dreh- Gleitlager) zwischen der äußeren Oberfläche des gerade ausgeformten Rohrbereichs (6) und der inneren Oberfläche des Adapterstücks (7), welche sich zueinander in einem leicht berührenden Oberflächenkontakt befinden, ausgebildet ist.

Die Länge des gerade ausgeformten Rohrbereichs (6) beträgt dabei vorteilhaft nahezu ein Drittel bis ein Zehntel der Länge des Krümmungsbereiches (4).

Die Länge des Adapterstückes (7) ist vorteilhaft nahezu doppelt so lang wie die Länge des gerade ausgeformten Rohrbereichs (6), so dass das Adapterstück (7) über den gerade ausgeformten Rohrbereich (6) und über die erste Öffnung (21) überragt.

Der Grad der Drehbarkeit des Adapterstückes (7) um die Drehachse -X-X- kann im Bedarfsfall durch Anschläge begrenzt sein.

Das Adapterstück (7) ist besonders vorteilhaft als Anschlusskonus mit 15 mm Durchmesser ausgeführt, um so die im Bereich der Trachealkanülen üblichen Anbauelemente, wie bspw. Beatmungsschläuche, gut mit dem Adapterstück (7) verbinden zu können.

Bei dem Trachealkanüleninnenrohr kann unmittelbar im Bereich des Übergangs des sich konisch aufweitenden Rohrbereichs (5) in den gerade ausgeformten Rohrbereich (6) in der Oberfläche des Rohrbereichs (5) eine umlaufende Nut (51) angeordnet sein. Vermittels dieser Nut (51) können entsprechend ausgestaltete Trachealkanülenaußenrohre befestigt werden. Dabei rastet die umlaufende Nut (51) in dafür vorgesehene Rastelemente des Trachealkanülenaußenrohrs ein (nicht in der Figur dargestellt).

Das Trachealkanüleninnenrohr besteht aus weichen oder halbstarren Kunststoff, welcher im medizinischen Bereich für Trachealkanülen üblich ist.

Der Vorteil dieses Trachealkanüleninnenrohrs besteht darin, dass eine freie Drehbarkeit zwischen dem Trachealkanüleninnenrohr und einem an dieses Rohr zu koppelnden Bauteils um die Drehachse -X-X- im Raum durch das drehbare Adapterstück ermöglicht wird, so dass bspw. ein mit dem Trachealkanüleninnenrohr verbundener Beatmungsschlauch entlastet wird und sich nicht verdrehen kann.
Durch diese Entlastung wird auch bei einer Dauerbelastung der Kopplungsstelle eine hohe mechanische Stabilität dieser Verbindung aufrechterhalten.

Alle in der Beschreibung, dem Ausführungsbeispiel und den nachfolgenden Ansprüchen dargestellten Merkmale können sowohl einzeln als auch in beliebiger Kombination miteinander erfindungswesentlich sein.

### Bezugszeichenliste

- 1 -: Kanüle
- 2 -: erstes freien Ende
- 21 -: erste Öffnung
- 3 -: zweite freien Ende
- 31 -: zweite Öffnung
- 4 -: bogenförmigen Krümmungsbereich
- 5 -: sich konisch aufweitender Rohrbereich
- 6 -: gerade ausgeformter Rohrbereich
- 61 -: Nut
- 7 -: hülsenförmiges Adapterstück
- 71 -: Wulst

- -X-X- -: Achse im Raum (Drehachse)

## Patentansprüche

1. Trachealkanüleninnenrohr zum Einführen in ein Trachealkanülenaußenrohr einer Trachealkanüle bestehend aus einer rohrförmigen Kanüle (1) mit einer ersten Öffnung (21) an einem ersten freien Ende (2) und einer zweiten Öffnung (31) an einem zweiten freier Ende (3), wobei die Kanüle (1) zwischen den beiden freien Enden (2 und 3) einen bogenförmigen Krümmungsbereich (4) aufweist, der Krümmungsbereich (4) am ersten freien Ende (2) in einen sich konisch aufweitenden Rohrbereich (5) übergeht und an den sich ein gerader Rohrbereich (6) anschließt, dessen Durchmesser größer ist als der Durchmesser des Tracheaikanüleninnenrohrs im Krümmungsbereich (4), **dadurch gekennzeichnet, dass** an dem geraden Rohrbereich (6) ein um seine Drehachse (-X-X-) um 360° unbegrenzt oder durch Anschläge begrenzt drehbar gelagertes, hülsenförmiges Adapterstück (7) angebunden ist, in dem der gerade Rohrbereich (6) in seiner äußeren Oberfläche eine umlaufende Nut (61) aufweist, in welche passgenau eine umlaufende Wulst (71) der inneren Oberfläche des Adapterstücks (7) eingreift, so dass ein durch Nut (61) und Wulst (71) geführtes Reiblager zwischen der äußeren Oberfläche des geraden Rohrbereichs (6) und der inneren Oberfläche des Adapterstücks (7), welche sich zueinander in einem leicht berührenden Oberflächenkontakt befinden, ausgebildet ist.

2. Trachealkanüleninnenrohr gemäß Anspruch 1, **dadurch gekennzeichnet, dass** die Länge des gerade ausgeformten Rohrbereichs (6) ein Drittel bis ein Zehntel der Länge des Krümmungsbereiches (4) beträgt.

3. Trachealkanüleninnenrohr gemäß Anspruch 1, **dadurch gekennzeichnet, dass** die Länge des Adapterstückes (7) doppelt so lang ist wie die Länge des gerade ausgeformten Rohrbereichs (6), so dass das Adapterstück (7) über den gerade ausgeformten Rohrbereich (6) und über die erste Öffnung (21) überragt.

4. Trachealkanüleninnenrohr gemäß Anspruch 3, **dadurch gekennzeichnet, dass** das Adapterstück (7) als Anschlusskonus mit 15 mm Durchmesser ausgeführt ist.

5. Trachealkanüleninnenrohr gemäß Anspruch 1, **dadurch gekennzeichnet, dass** sich im Bereich des Übergangs des sich konisch aufweitenden Rohrbereichs (5) in den gerade ausgeformten Rohrbereich (6) in der Oberfläche des Rohrbereichs (5) eine umlaufende Nut (51) befindet, vermittels derer ein Trachealkanülenaußenrohr befestigbar ist.

6. Trachealkanülcninnenrohr gemäß einen oder mehrerer der voran stehenden Ansprüchen 1 bis 5, **dadurch gekennzeichnet, dass** Trachealkanüleninnenrohr aus einem weichen oder halbstarren Kunststoff besteht.

## Claims

1. A tracheal cannula inner tube to be inserted into a tracheal cannula outer tube of a tracheal cannula, comprising a tubular cannula (1) with a first opening (21) at a first free end (2) and a second opening (31) at a second free end (3), wherein the cannula (1) has an arch-shaped curved region (4) between the two free ends (2 and 3), the curved region (4) changes at the first free end (2) into a conically expanding tube region (5) with a subsequent straight tube region (5) the diameter of which is larger than the diameter of the tracheal cannula inner tube in the curved region (4), **characterized in that** a pivoting, sleep-shaped adapter piece (7) which has an unlimited rotation capability of 360° around its rotating axis (-X-X-) or a rotation capability limited by stop units is attached to the straight tube region (6) and in which the outer surface of the straight tube region (6) is provided with a circumferential groove (61) into which a circumferential bead (71) of the inner surface of the adapter piece (7) engages in an accurately fitting manner such that a friction bearing is formed by the groove (61) and bead (71) between the outer surface of the straight tube region (6) and the inner surface of the adapter piece (7) which are in a slight surface contact with each other.

2. The tracheal cannula inner tube according to claim 1, **characterized in that** the length of the straight tube region (6) is one third to one tenth the length of the curved region (4).

3. The tracheal cannula inner tube according to claim 1, **characterized in that** the length of the adapter piece (7) is double the length of the straight tube region (6) so that the adapter piece (7) protrudes the straight tube region (6) and the first opening (21).

4. The tracheal cannula inner tube according to claim 3, **characterized in** the adapter piece (7) is designed as a connection cone with a diameter of 15 mm.

5. The tracheal cannula inner tube according to claim 1, **characterized in that** in the region of the transition of the conically expanding tube region (5) to the straight tube region (6) a circumferential groove (51), which can be used to attach a tracheal cannula outer tube, is provided in the surface of the tube region (5).

6. The tracheal cannula inner tube according to one of the previous claims 1 to 5, **characterized in that** the tracheal cannula inner tube is made of soft or semirigid plastic material.

## Revendications

1. Tube intérieur de sonde trachéale destiné à être introduit dans le tube extérieur d'une sonde trachéale se composant d'une sonde tubulaire (1) ayant un premier orifice (21) à une première extrémité libre (2) et un deuxième orifice (31) à une deuxième extrémité libre (3), ladite sonde tubulaire (1) possédant une partie coudée (4) en courbe entre les deux extrémités libres (2 et 3) et la partie coudée (4) se raccordant au niveau de la première extrémité libre (2) à une partie tubulaire (5), qui s'évase en forme de cône, suivie immédiatement d'une partie tubulaire rectiligne (6) dont le diamètre est plus grand que celui de la partie coudée (4) du tube intérieur de sonde trachéale, est **caractérisé en ce qu'**un élément adaptateur (7) en forme de manchon, supporté de manière à pouvoir tourner sur 360° de façon illimitée ou limitée par des butées autour de son axe de rotation -X-X- est relié à la partie tubulaire rectiligne (6) et que la surface extérieur de la partie tubulaire rectiligne (6) est munie d'une gorge périphérique (61), formant ainsi un palier à friction, guidé par la gorge (61) et le bourrelet (71), entre la surface extérieure de la partie tubulaire rectiligne (6) et la surface intérieure de l'élément adaptateur (7), qui sont en léger contact superficiel l'un avec l'autre.

2. Tube intérieur de sonde trachéale suivant la revendication 1 est **caractérisé en ce que** la longueur de la partie tubulaire rectiligne (6) est d'un tiers à un dixième de la longueur de la partie coudée (4).

3. Tube intérieur de sonde trachéale suivant la revendication 1 est **caractérisé en ce que** la longueur de l'élément adaptateur (7) est deux fois plus long que la longueur de la partie tubulaire rectiligne (6) de sorte que l'élément adaptateur (7) dépasse la partie tubulaire rectiligne (6) et le premier orifice (21).

4. Tube intérieur de sonde trachéale suivant la revendication 3 est **caractérisé en ce que** l'élément adaptateur (7) est réalisé comme cône de raccordement avec un diamètre de 15 mm.

5. Tube intérieur de sonde trachéale suivant la revendication 1 est **caractérisé en ce qu'**une gorge périphérique (51) se trouve dans la zone de la transition de la partie tubulaire (5), qui s'évase en forme de cône, à la partie tubulaire rectiligne (6) dans la surface de la partie tubulaire (5) et un tube extérieur d'une sonde trachéale peut être fixé au moyen de cette gorge périphérique.

6. Tube intérieur de sonde trachéale suivant une ou plusieurs des revendications précédentes 1 à 5 est **caractérisé en ce que** le tube intérieur de sonde trachéale se compose d'une matière plastique souple ou semi-rigide.
